# EUROPEAN PATENT APPLICATION

(11) **EP 4 582 120 A1**
(43) Date of publication of application: **09.07.2025**
(21) Application number: 24150527.0
(22) Date of filing: 05.01.2024
(51) Int. Cl.: A61M 5/20, A61M 5/24, A61M 5/315

(54) **SAFETY MECHANISM FOR A REUSABLE AUTOINJECTOR**

(71) Applicant: Ypsomed AG, 3401 Burgdorf (CH)
(72) Inventor: Hostettler, Patrick, 3415 Hasle (CH); Scheurer, Simon, 3006 Bern (CH)
(74) Representative: Kleiner, Stefan

(57) **Abstract**

The present invention relates to a safety mechanism for a drug delivery device (1). The safety mechanism comprising a reservoir unit (2) adapted to hold a reservoir (5) and a drive unit (3) releasably attachable to the reservoir unit (2). The drive unit (3) comprises a housing (10) defining a longitudinal axis, a drive (91) arranged inside the housing for driving a plunger rod (40) and a movable arm (70) that can be in a radially inner locking position, in which the reservoir unit (2) is locked relative to the drive unit (3) and wherein the arm (70) can be in a radially outer release position in which the reservoir unit (2) is released from the arm (70). The mechanism further comprises a trigger member (50) movable along the longitudinal adapted to move the arm (70) between the release position and the locking position.

## Description

### FIELD OF THE INVENTION

The present invention relates to a safety mechanism for an injection device for injecting, delivering, administering, infusing or dispensing substances and/or liquids such as insulin, hormone preparations or vaccines. The safety mechanism comprises a reservoir unit adapted to hold the reservoir and a drive unit releasably attachable to the reservoir unit. The drive unit comprises a housing, a drive arranged inside the housing for dispense the liquid drug and a movable arm that can be in a radially inner locking position, in which position the reservoir unit is hold relative to the drive unit by the arm and wherein the arm can be in a radially outer release position in which the reservoir unit is released from the arm.

### BACKGROUND OF THE INVENTION

A variety of diseases exist that require regular treatment by subcutaneous administration of a medicament, and several drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism in order to move the plunger of the syringe upon actuation of the autoinjector. The drive mechanism typically comprises a source of drive, such as an electric motor or a mechanical strong spring for moving a transfer element, for example a rod, which acts on the plunger of the syringe. The prefilled syringe has a needle or cannula that is permanently attached to a first end of a syringe barrel or reservoir that is sealed at the opposing end by the plunger.

For safety and hygiene reasons it is desirable that the needle does not protrude from a housing of the autoinjector except for the time when the needle is used for injection of a medicament. Thus, either the autoinjector moves the needle out of the housing for the injection and back into the housing after injection or the autoinjector includes a needle cover which may be moved to unsheathe the needle for injection and which may be moved back to a needle covering position after the injection.

The majority of autoinjectors are configured as single use devices which incorporate both the syringe and the drive mechanism in the same housing. Such devices are usually disposable for hygiene reasons.

Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy.

In order to account for a need to handle and dispose the autoinjector parts differently semi-reusable autoinjectors have been developed. Such autoinjectors typically comprise a reusable drive unit as well as a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection.

WO2019191104 A1 discloses a reusable autoinjector comprising a disposable syringe unit holding a prefilled syringe and a reusable drive unit. The drive unit comprises a holding arm and a spring biased back plate. When the syringe unit is inserted into an opening in the drive unit a locking surface on a free end of the holding arm engages a ledge in syringe unit to lock the syringe unit in place in the drive unit.

US 2021/0038817 A1 discloses a reusable autoinjector with a disposable syringe unit and a reusable drive unit. The syringe unit includes a flexible member comprising a ledges adapted to be inserted into a circular groove in an opening of the drive unit. The ledges and the groove provide a snap-locking to secure the syringe unit to the drive unit.

EP 3639871 A1 discloses a reusable autoinjector with a drive unit including an electric motor. The syringe unit comprises two oppositely positioned rearwardly directed tongues having outwardly extending protrusions. These protrusions are arranged to fit into oppositely positioned recesses on an inner surface of a cavity in the drive unit. After insertion of syringe unit the tongues with the protrusions in the recesses are locked in this position by a locking sleeve that is pushed forward so that it comes in contact with the inner surfaces of the tongues, thereby locking them in the radial direction.

### DESCRIPTION OF THE INVENTION

It is an objective of the invention to provide a reliable and releasable connection between a syringe unit and a drive unit of an injection device.

This objective is achieved by a safety mechanism, an injection device or a method according to the independent claims. Preferred embodiments are evident from the dependent claims.

The invention relates to a safety mechanism for a drug delivery device for dispensing a liquid drug from a reservoir through an injection needle. The safety mechanism comprises
- a reservoir unit adapted to hold the reservoir;
- a drive unit releasably attachable to the reservoir unit. The drive unit comprises a housing defining a longitudinal axis and a drive arranged inside the housing for driving a plunger rod to dispense the liquid drug. The drive unit further comprises a movable arm that can be in a radially inner locking position, in which position the reservoir unit is held and locked relative to the drive unit by the arm and the arm can be in a radially outer release position in which position the reservoir unit is released from the arm and the arm does not engage the reservoir unit such that the reservoir unit can be removed from the drive unit.

The safety mechanism further comprises a trigger member movable along the longitudinal axis relative to the housing. The trigger member can cooperate directly or indirectly (via intermediate member) with the movable arm such that a movement of the trigger member along the longitudinal axis causes the arm to move between the release position and the locking position. Preferably, a movement of the trigger member in a first longitudinal direction causes the arm to move from the release position to the locking position and a movement of the trigger member in a second longitudinal direction (counter to the first direction) causes the arm to move from its locking position to its release position.

That means the arm can be moved between the locking position and the release position by a longitudinal movement of the trigger member. In other words, the reservoir unit can be locked to the drive unit or released from the drive unit by axially moving the trigger member. The trigger member can be moved manually by a user force or by an automatic drive such as, for example, a motor or biased elastic member.

The actuation of the arms by the trigger member allows for a compact design as the trigger member moves (preferably moves exclusively) along the longitudinal axis of the device and preferably does not move radially and hence does not need any space in radial direction.

Furthermore, the movable arm provides a safe and reliable locking of the reservoir unit to the drive unit such that the reservoir unit cannot be separated unintentionally from the drive unit.

The arm may be coupled directly or indirectly to the trigger member, for example, by joint mechanics, a toggle link or by a cam control such that the linear (axial) movement of the trigger member is transferred to a (partially) radial movement of the arm. The arm may be moved between the release position and the locking position by deflection, rotation or by shifting or the arm may be moved by a combination of these movements.

In the locking position the arm can engage the syringe unit and hold the syringe unit in place such that it cannot be pulled out of the drive unit. That means in the locking position the syringe unit and the drive unit are coupled or locked together. In the release position the reservoir unit is released from the arm and thus it can be pulled out of the drive unit by the user.

The trigger member may be an elongated member, a connecting member or a sleeve that can cooperate with the arm or is coupled to the arm. The trigger member may be designed such that it connects the drive and the movable arm.

Preferably, the trigger member is guided within the housing by a linear guide of the housing enabling exclusively a movement along the longitudinal axis and preventing a movement in a radial or angled directions.

The drug delivery device may be either a manually actuated or an automatic delivery device. Automatic devices or autoinjectors typically include automatic drive means such as an elastic element (for example a pre-tensioned spring) or an electric motor to drive a dispensing member to dispense the liquid drug form the reservoir. Manual delivery devices include a manual drive such as a dispensing button that has to be pressed by the user and by a human force to move the dispensing member in dispensing direction.

The delivery device is preferably an injection device and more preferably an autoinjector including a reservoir in form of a prefilled syringe. The prefilled syringe has a needle or cannula that is permanently attached to a first end of a syringe barrel or reservoir that is sealed at the opposing end by a plunger. The autoinjector is adapted to dispense the whole content of the syringe in a single injection stroke. Alternatively, the reservoir may be a cartridge with a connecting portion at the distal end adapted to be connected to an injection needle prior injection.

The safety mechanism comprises the above-mentioned components and may comprise further components such as a dispensing mechanism or/and a needle cover for example, to prevent unintentional access to an injection needle.

The reservoir unit with the reservoir is not operational without the drive unit. To prepare the delivery device for an administration the user has to attach or couple the reservoir unit to the drive unit. The drive unit includes the drive adapted to dispense the liquid drug from the reservoir hold inside the reservoir unit when the reservoir unit is attached to the drive unit.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the trigger member is U-shaped or sleeve-shaped and the reservoir unit is at least partially inside the sleeve-shaped trigger member when the reservoir unit is attached to the drive unit. The U-shaped or sleeve-shaped trigger member enables a compact design as the components can be arranged inside the sleeve.

Alternatively, the trigger member is an elongated arm, lever or cantilever coupled to the radially movable arm.

The trigger member preferably cooperates with the arm by a cam control (sliding block guide) which comprises a cam and a groove adapted to guide the cam along a predefined track. The trigger member may include the cam or the groove and the arm may include the other of the cam and the groove. That means a movement of the arm is restricted to a predefined motion path or track as the cam is guided within the groove. Furthermore, a transmission ration can be achieved by the cam control such that, for example, a trigger member movement is transferred to a shorter or longer movement of the arm. Alternatively, the trigger member may be coupled to the arm, for example, by a mechanical joint or a gear wheels.

Preferably, the cam protrudes in the direction perpendicular to the longitudinal axis. Alternatively, the cam protrudes in the direction of a (radial) movement of the arm.

In a preferred embodiment the arm includes a connecting end and a free end, wherein the free end comprises a radially inward directed protrusion. The reservoir unit comprises preferably a lateral opening or window which enables the arm to be inserted to engage the reservoir and thus lock the reservoir to the drive unit. Preferably, the arm can be inserted between a proximal end of a needle shield (preferable a rigid needle shield or RNS) of the reservoir and a distal end of a reservoir body when the arm is in the locking position.

When the arm is in its locking position the protrusion may be located at the distal end of the reservoir and thus preventing the reservoir from being moved distally out of the drive unit when the syringe unit is attached to the drive unit.

Moreover, the protrusion between the reservoir and the needle shield may help to separate the needle shield from the reservoir, for example, prior injection when the needle shield has to be removed from the injection needle by the user and hence the sterile barrier is removed.

The connecting end of the arm is preferably pivotable supported by the housing such that the arm can rotate. That means when the trigger member is moved relative to the housing along the longitudinal axis the arm may be rotated but is not linearly moved relative to the housing.

The arm may have an elongated shape and/or it may have different portions. Preferably, the arm comprises a radially inwards protruding engagement portion and a longitudinal portion wherein the engagement portion and the longitudinal portion are arranged essentially perpendicular to each other. Thus, in this embodiment the arm has a L-shaped form.

In a preferred embodiment the drive unit includes a carrier adapted to engage directly a proximal rim or flange of the reservoir to connect the reservoir unit to the drive unit. That means the reservoir unit is coupled to the drive unit by means of the carrier which (preferably exclusively) holds the rim or flange of the reservoir of the reservoir unit. The carrier preferably holds the reservoir in a releasable manner such that the reservoir unit cannot fall off. However, the reservoir unit when held by the carrier is not yet locked to the drive unit and can be removed from the drive unit if the user pulls the reservoir unit.

The coupling between the reservoir and the carrier may be implemented, for example, by clamps, holding arms or snap-fit elements allowing to releasably couple or attach the carrier to the reservoir and namely to a flange or rim of the reservoir.

The carrier is preferably movable along the longitudinal axis relative to the housing between an extended position and a retracted position. The carrier may move the reservoir, for example, to bring the reservoir in a position in which the needle is covered to prevent unintentional access to the injection needle. Furthermore, the reservoir may be moved by the carrier to the extended position in order to insert the injection needle into injection site. As the reservoir is hold inside the reservoir unit the reservoir unit is moved too by the carrier relative to the housing.

The carrier may be manually moved by a user force or automatically by a biased elastic member or by an electric drive. The housing may have a corresponding guide, preferably a linear guide providing a guiding for the carrier along the longitudinal axis.

In a preferred embodiment the mechanism comprises further a carrier spring member adapted to move the carrier relative to the housing. That allows for an easy and reliable movement of the carrier together with the reservoir. In particular, the spring member may be used to puncture or insert an injection needle of the reservoir into the injection site. The spring member is preferably biased when the carrier is in the retracted position. By way of example, the spring member may be a mechanical spiral spring or an elastomer that can be compressed.

Preferably, the drive of the drive unit is an electric motor adapted to move the plunger rod in dispensing direction. The drive unit may further include a controller to drive and control the electric motor. The controller and the motor may be part of an electronic unit inside the drive unit.

Alternatively, the drive may be a biased elastic member such as a mechanical spring or an elastomer element.

The trigger member is preferably operatively coupled to the motor. That means the trigger member may be directly connected to a rotating motor member or the trigger member may be coupled to the motor by an intermediate member, such as a lever, a connecting arm, a toothed wheel or a gear.

When the motor rotates in a first rotational direction the trigger member is moved in the first direction along the longitudinal axis (preferably in proximal direction) and when the motor rotates in a second, counter rotational direction the trigger member is moved in the second direction (preferably in distal direction) along the longitudinal axis.

A gear such as a worm gear may be used to transfer the rotational movement of the motor into a linear movement of the trigger member. This allows to control the trigger member movement by the motor and thus the switching of the arm between its release position and its locking position and thus the locking or unlocking of the reservoir unit.

Additionally, the carrier may be operatively coupled to the motor. That means the carrier is movable along the longitudinal axis relative to the arm by the electric motor. In this case the motor not only drives the plunger rod for dispensing but also the carrier is moved by the motor. This may be achieved, for example, by a coupling of the carrier to a motor drive shaft or to the plunger rod.

In a preferred embodiment the reservoir unit comprises only one single component, namely a holding member adapted to envelop and support the reservoir. Hence, the reservoir is held by the holding member but is still able to move relative to the holding member. The holding member may provide needle cover for the injection needle of the reservoir. The reservoir is thus preferably movable along the longitudinal axis relative to the holding member between a needle covering position and a retracted position in which the needle of the reservoir protrudes from the holding member.

That means one component can fulfill a plurality of functions. The holding member holds the reservoir in place and the holding member may further provide a needle cover depending on the position of the reservoir within the holding member. The holding member may include a guide (for example guiding rails or grooves) for the reservoir to enable and to guide a linear movement of the reservoir relative to the holding member.

The holding member is preferably sleeve-shaped and the cross-section may be rectangular, circular or elliptical shaped.

In a preferred embodiment the reservoir unit comprises a holding element for holding the reservoir and wherein the housing or an insert fixedly connected to the housing comprises a release element adapted to engage the holding element to release the reservoir upon contact of the holding element with the release element. That means after the release the reservoir is free to move and can be moved by the carrier, in particular between a retracted position and an extended position. The reservoir unit is preferably moved relative to the housing of the drive unit until the release element encounters a counter element of the housing releasing the reservoir such that the reservoir can move, for example, from the retracted position to the extended position to insert the injection needle into the injection site.

The release element may be, for example, a cam, knob or arm. Preferably, the release element is a sloped surface and the holding element comprises preferably a counter sloped surface which is arranged such that when the holding member is moved in towards a retracted position the sloped surface and the counter sloped surface engage each other and slide along each other thereby releasing the reservoir from the holding element. Subsequently and after release, the reservoir may be moved relative to the reservoir holder.

The invention relates further to a drug delivery device comprising the safety mechanism as described above. The reservoir unit is preferably a disposable syringe unit adapted to hold or support the reservoir which is preferably a prefilled syringe. In this embodiment the drive unit is preferably a reusable unit adapted to be releasably attached to the syringe unit. The syringe includes the injection needle which is permanently and non-removably attached to a distal end of the syringe.

In this embodiment the delivery device is an injection device and in particular a reusable or semi-reusable autoinjector (also named as semi-disposable autoinjector) comprising the reusable drive unit and the disposable syringe unit including the syringe with the drug.

The invention relates further to a method for connecting a reservoir unit to a drive unit of a drug delivery device for dispensing a liquid drug from the reservoir, the method comprising the steps of
a) Inserting, a proximal end of the reservoir unit into an opening in the drive unit;
b) Moving a trigger member of the drive unit along a longitudinal axis relative to a drive unit housing;
c) Moving an arm of the drive unit, by the trigger member movement, from a radially outer release position to a radially inner locking position, in which position the reservoir unit is held and locked relative to the drive unit by the arm.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts an exploded view of an autoinjector according to the invention;
- Fig. 2: depicts a sectional view of a syringe unit separated from a drive unit of the autoinjector;
- Fig. 3: depicts a sectional view when the syringe unit is inserted into the drive unit;
- Fig. 4: depicts a sectional view when the syringe unit is in a retracted position inside the drive unit;
- Fig. 5: depicts a sectional view in a plane 90° rotated to the plane of figure 4 of the autoinjector after a push-on-skin;
- Fig. 6: depicts a sectional view when the syringe is in an inserted position and
- Fig. 7: depicts a sectional view after the injection when a needle cover lock is active.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

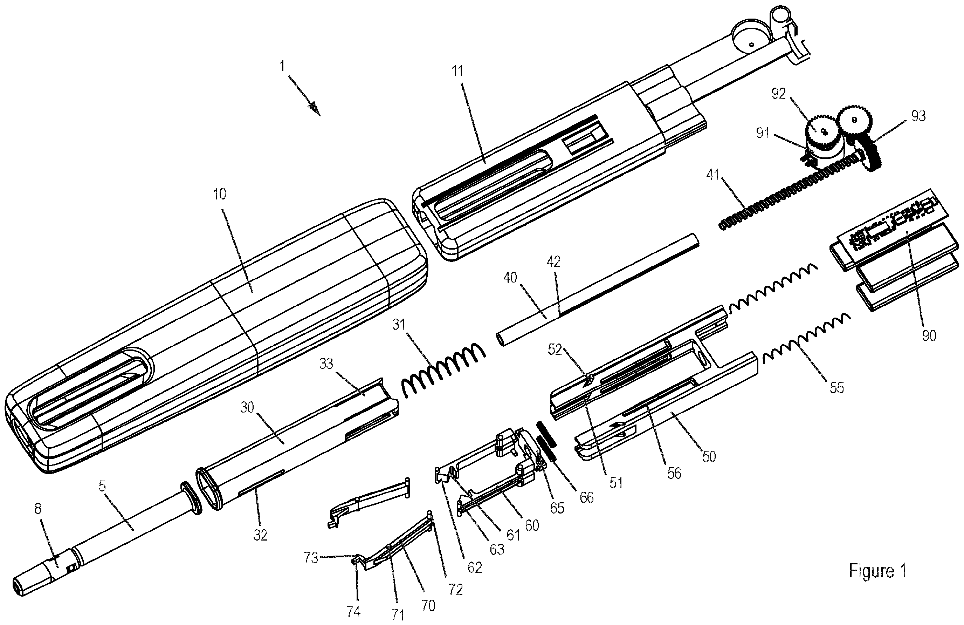

Figure 1 shows a perspective view of a drug delivery device of the invention in form of a semi-reusable autoinjector 1. The autoinjector 1 comprises a reservoir unit in form of a disposable syringe unit 2 and a reusable drive unit 3. As shown in figure 1 the drive unit 3 has an elongated housing 10 defining a longitudinal axis. The drive unit 3 includes an opening or cavity adapted to accommodate a proximal portion of the syringe unit 2 to releasably attach or couple the syringe unit 2 to the drive unit 3.

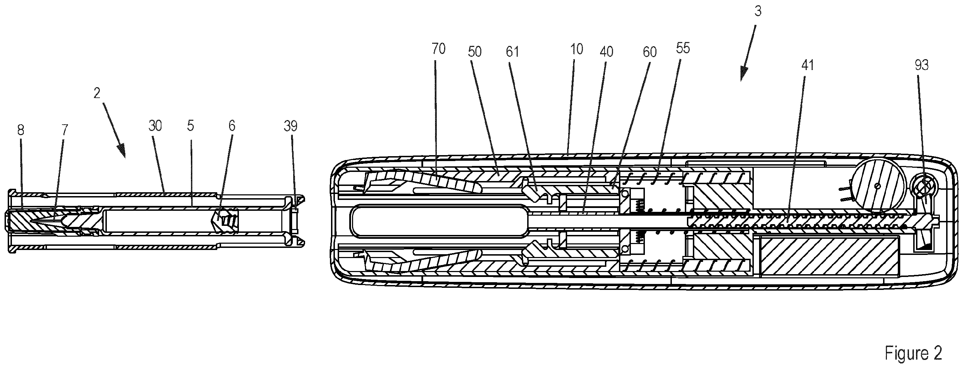

Figure 2 depicts a sectional view of the syringe unit 2 and the drive unit 3 in a separated state. The syringe unit 2 includes only one injection molded plastic in the form of a sleeve-shaped syringe holder 30 (reservoir holder) providing a support for the prefilled syringe 5 and additionally provides a needle cover as described in detail below. The syringe unit 2 comprises further the prefilled syringe 5 including a barrel made of glass and a piston 6 inside the barrel sealing the content within the barrel. On a distal end of the barrel an injection needle 7 is permanently connected. A rigid needle shield (RNS) 8 is initially mounted on the injection needle. The syringe 5 is cinched within the syringe holder to prevent a movement in proximal direction by two retaining cams 39, shown in figure 2. In an outer wall the syringe holder has two oppositely located openings 32 or windows enabling access to the syringe from outside.

The drive unit 3 comprises the housing 10, an insert 11 fixedly connected to the housing, a trigger member 50 movable along the longitudinal axis relative to the insert (and hence relative to the housing), two oppositely arranged and pivotably supported holding arms 70, a movable carrier 60, a plunger rod 40 adapted to engage the piston 6 within the syringe 5, a threaded rod 41 in threaded connection with the plunger rod 40 and an electronic unit 90 including an electric motor 91 and a controller. The motor 91 is connected to a gear wheel 92 which is connected to a worm gear 93 to transmit a motor movement to the threaded rod 41.

Each holding arm 70 comprises a pivot 72 to enable a rotation. Furthermore, each holding arm 70 comprise a cam 71 and the trigger sleeve 50 includes corresponding guiding tracks 51 (see figure 1) adapted to accommodate and guide the cams 71 within the tracks 51 when the trigger sleeve 50 moves relative to the holding arms 70. The cams 71 and guide track 51 thus form a cam control.

The carrier 60 comprises two arms which include on their free end a carrier cam 63 accommodated in corresponding grooves 56 in the trigger member 50, see figure 1. Two oppositely arranged trigger springs 55 bias the trigger member in a distal direction. Similarly, a syringe holder spring or carrier spring 31 biases the syringe holder 30 in a distal direction when the syringe unit is connected to the drive unit.

As it can be seen in figure 2 in a distal portion of the housing 10 the opening for accommodating the syringe unit 2 and the trigger sleeve 50 and the holding arms 70 are located. The carrier 60 is arranged inside a proximal portion of the trigger member 50. The electronic unit 90 (figure 1) is located in a proximal portion within the housing 10.

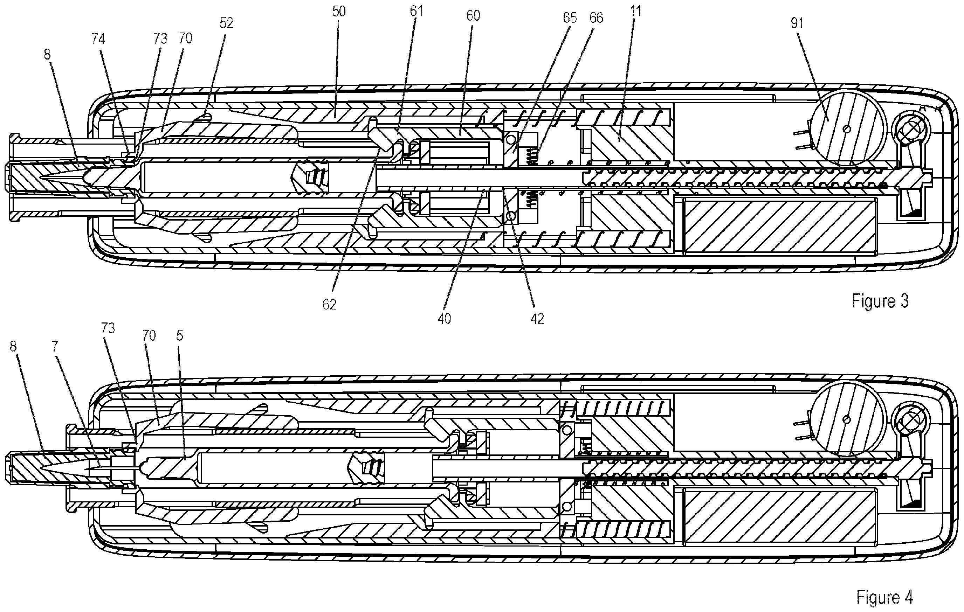

Figure 3 depicts a state when the syringe unit 2 is inserted into the drive unit opening. In the state shown in figure 3 the syringe unit 2 is held in place by the carrier 60 but is not yet locked to the drive unit 3.

The carrier 60 includes two oppositely arranged arms which are connected by a connector element 65. At their free ends each arm comprises a holding element in form of a clamp 61. The clamps 61 are pivotably supported by the insert 11 and springs 66 bias the clamps 61 in an initial nondeflected position. The clamps 61 are adapted to engage a proximal rim 9 of the syringe to hold the syringe 5 in place. Each clamp 61 includes on a distal end a sloped or angled release surface 62 which allows to radially deflect the clamps 61 when a user inserts the syringe unit 2 into the drive unit 3. The cams 63 can travel within grooves 56 (see figure 1) to enable this deflection. After this insertion the clamps 61 deflect back into their initial position and engage the syringe rim 9.

When the syringe unit 2 is inserted into the drive unit 3 as shown in figure 3 a tag reader reads a tag on an outside of the syringe holder 30 and the electronic unit 90 of the drive unit 3 transmits the read data to an external device for verification. If the verification is successful a controller in the electronic unit receives a command to continue with the injection process. That means the controller drives the motor 91 in a rearward direction meaning that the plunger rod 40 is moved in rearward proximal direction. As the plunger rod 40 comprises a radial ledge 42 engaging protrusions in the connector 65 the carrier 60 latter is moved proximally together with the plunger rod 40 a short distance. The trigger member 50 connected to the carrier via cam 63 and grooves 56 is moved too and hence the trigger member 50 moves relative to the holding arms 70. That means the cam 71 travel within the guiding tracks 51. When the trigger member 50 is in an initial distal position the cams 71 are an in angled portion 52 of the guiding tracks 51 (see figure 1 and 3). When the trigger member 50 moves proximally the angled portion 52 forces the cams 71 in a radially inward direction meaning that the arms 70 are forced to move from a radially outer release position to a radially inward locking position. That in turn means a radially inwards directed protrusion 73 of an engagement portion of each arms enters the lateral openings 32 in the syringe holder. When the arms 70 are in their locking position the protrusions 73 are located in between a distal end of the syringe barrel and a proximal end of the RNS 8 as shown in figure 3. In more detail, an axial portion 74 of each arm 70 abuts a distal outer surface of the RNS 8 and the radial protrusion 73 of each arm 70 engages a proximal end surface of the RNS 8. The arms 70 may be made of a flexible material such that the radial protrusions 73 can be pressed between the proximal end of the RNS and the distal end of the syringe barrel and a mechanical play or tolerances may be compensated by the flexibility of the arms.

In the state shown in figure 3 the syringe unit 2 is locked to the drive unit 3 by the holding arms and cannot be pulled out.

Figure 4 depicts a next state when the syringe unit 2 is in a retracted position. When the presence of a syringe unit is sensed by a sensor inside the drive unit the controller further drives the motor 91 in rearward direction to retract the syringe unit. Thus, the plunger rod 40 and the carrier 60 which is connected to the plunger rod via ledge 42 and connector 65 and biased to the plunger rod by spring 31 move proximally too. The trigger springs 55 get compressed during the proximal movement. That means the whole syringe unit 2 moves relative to the holding arms 70. As the protrusions 73 engage the proximal end of the RNS 8 the latter is prevented from being moved proximally and is shifted along the injection needle 7 until the injection needle is pulled out of the RNS 8 and is located proximally to the needle, as shown in figure 4. In other words, the holding arms 70 hold the RNS 8 in place while the syringe 5 is moved proximally to separate the RNS 8 and the syringe 5 and hence the sterile barrier is removed. The user can now remove the RNS 8 from the syringe unit 2 and the autoinjector is then ready for an injection.

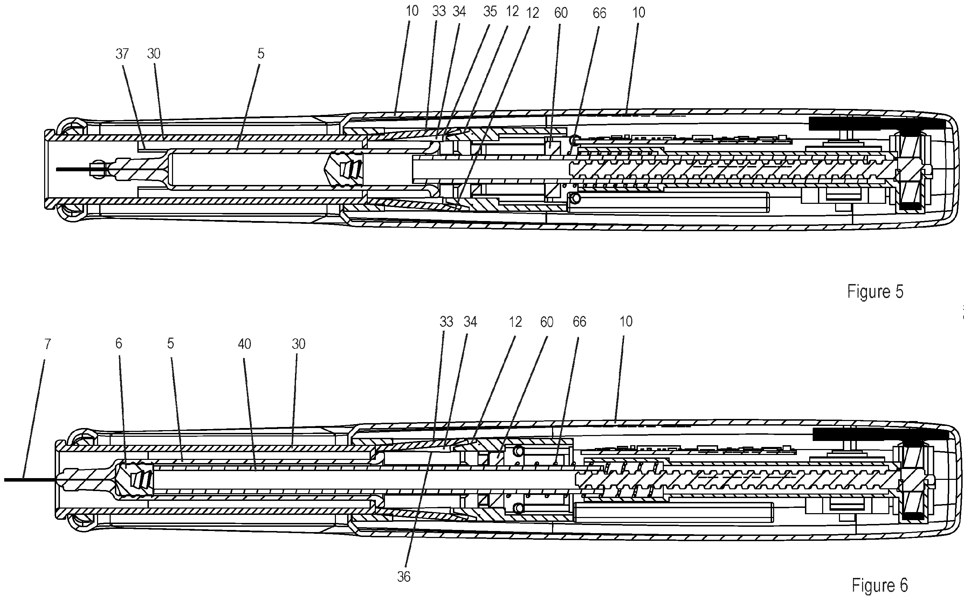

To start the injection the user places a distal end of the syringe holder 30 onto the injection site and pushes the autoinjector 1 against the injection site (push on skin). The syringe holder 30 (which acts as needle cover sleeve) is thereby moved in proximal direction relative to the housing 10. This state is shown in figure 5 which depicts a sectional view of the autoinjector wherein the plane of the sectional view is 90° rotated compared to the plane of the sectional view of figures 2 to 4.

The syringe holder 30 comprises on a proximal end two oppositely arranged radially deflectable elements 33 which have on their inner side a first sloped surface 38. During retraction of the syringe holder 30 these first sloped surfaces 38 of the elements 33 are moved onto second sloped surfaces 12 of the insert 11 which causes the elements 33 to deflect outwards. The element with the sloped surface 12 is positioned between the retaining cams 39 (see figure 2) of the syringe holder. This means a radially inwards protruding rib 34 with a stop surface 35 of the element 33 is moved radially outward too and thus out of engagement with a rim 9 of the syringe 5. That in turn means the syringe 5 is free to move in distal insertion direction relative to the syringe holder 30.

When the syringe holder 30 is in a retracted position a switch in the drive unit is activated and generates a signal for the controller to start the injection. The controller drives the motor 91 and the threaded rod 41 is rotated in forward direction. Due to the biased spring 31 the carrier 60 moves proximally too. The threaded rod 41 may have a varying thread pitch (thread lead) such that the insertion speed is increased compared to the subsequent injection speed.

The syringe 5 moves then together with the carrier 60 relative to the syringe holder 30 in an injection position as shown in figure 6. This syringe movement is guided by guiding rails 37 on an inside of the syringe holder 30. In a distal end position of the syringe the injection needle 7 protrudes from the syringe holder 30 as shown in figure 6. The motor 91 further moves the plunger rod 40 distally such that the piston 6 inside the syringe barrel is moved distally and thus the drug is dispensed from the syringe 5 until the piston 6 reaches a distal end position as shown in figure 6.

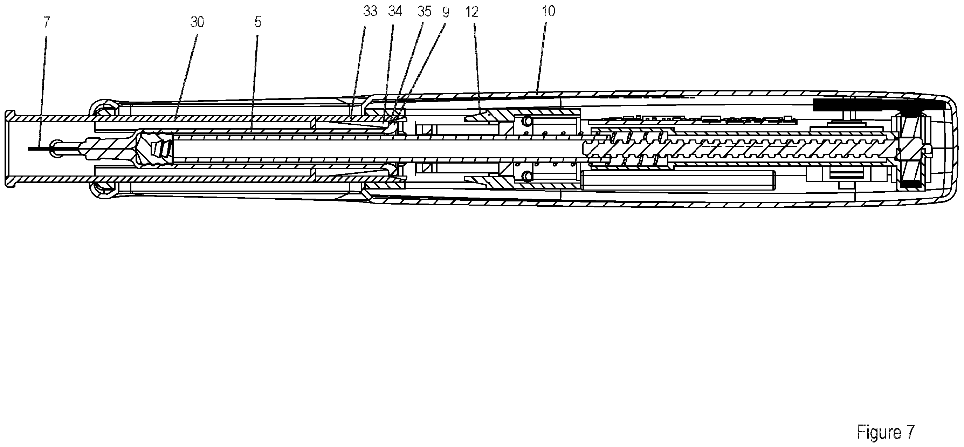

When the injection is completed, the controller issues a notification to the user that the autoinjector 1 can be removed from the injection site. When the autoinjector is removed from the injection site the syringe holder 30 ist moved distally by the biased trigger springs 55 such that it covers the injection needle 7. During this distal movement of the syringe holder 30 the first sloped surfaces 38 of the deflectable elements 33 get out of contact with the second sloped surfaces 12 and thus the elements 33 deflect radially inwards again. During distal movement the rib 34 can pass the syringe rim 9 due to its an angled surface 36 (figure 6). When the syringe holder 30 is in a distal covering position the stop surface 35 is positioned distally to the syringe rim 9 as shown in figure 7. The syringe holder 30 is thus prevented from being moved back in proximal direction and thus the syringe holder 30 is locked in its distal end position.

Figure 7 depicts this needle cover lock state. As shown in figure 7 the syringe holder 30 is in a distal end position covering the injection needle 7.

As a last step the trigger member 50 is moved back in a distal end position by the biased trigger springs 55. That means the angled portion 52 of the guiding tracks 51 forces the cams 71 radially outwards and thus the arms 70 move from the locking position to the radially outer release position. The syringe unit 2 is then no longer locked to the drive unit 3 and hence the user can remove the emptied syringe unit 2 from the drive unit 3. The latter is then ready to be loaded with a new syringe unit.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | autoinjector | 50 | trigger member |
| 2 | syringe unit | 51 | guiding tracks |
| 3 | drive unit | 52 | angled portion |
| 5 | syringe | 55 | trigger spring |
| 6 | piston | 56 | grooves |
| 7 | injection needle | | |
| 8 | RNS | 60 | carrier |
| 9 | rim | 61 | clamps |
| | | 62 | release surface |
| 10 | hosing | 63 | cam |
| 11 | insert | 65 | connector |
| 12 | second sloped surface | 66 | spring |
| 30 | syringe holder | 70 | holding arms |
| 31 | spring | 71 | cam |
| 32 | openings | 72 | pivot |
| 33 | deflectable elements | 73 | protrusion |
| 34 | rib | 74 | axial portion |
| 35 | stop surface | | |
| 36 | angled surface | | |
| 37 | guiding rails | 90 | electronic unit |
| 38 | first sloped surface | 91 | motor |
| 39 | retaining cam | 92 | gear wheel |
| | | 93 | worm gear |
| 40 | plunger rod | | |
| 41 | threaded rod | | |
| 42 | ledge | | |

## Claims

1. A safety mechanism for a drug delivery device (1) for dispensing a liquid drug from a reservoir (5) through a needle (7), the safety mechanism comprising a reservoir unit (2) adapted to hold the reservoir (5) and a drive unit (3) releasably attachable to the reservoir unit (2), the drive unit (3) comprising
- a housing (10) defining a longitudinal axis;
- a drive (91) arranged inside the housing for driving a plunger rod (40) to dispense the liquid drug;
- a movable arm (70) that can be in a radially inner locking position, in which the reservoir unit (2) is locked relative to the drive unit (3) and wherein the arm (70) can be in a radially outer release position in which the reservoir unit (2) is released from the arm (70),
**characterized in that** the mechanism further comprises a trigger member (50) movable along the longitudinal axis relative to the housing and adapted to cooperate with the movable arm (70) such that a movement of the trigger member along the longitudinal axis causes the arm (70) to move between the release position and the locking position.

2. Safety mechanism according to claim 1, wherein the trigger member (50) is U-shaped and wherein the reservoir unit (2) is at least partially inside the U-shaped trigger member 50 when the reservoir unit (2) is attached to the drive unit (3).

3. Safety mechanism according to claim 1 or 2, wherein the trigger member (50) can cooperate with the arm (70) by a cam control comprising a cam (71) and a groove (51) adapted to guide the cam (71) along a predefined track and wherein the trigger member (50) includes the cam (71) or the groove (51) and the arm (70) includes the other of the cam (71) and the groove (51).

4. Safety mechanism according to any of claims 1 to 3, wherein the arm (70) includes a connecting end and a free end, wherein the free end comprises a radially inward directed protrusion (73) and wherein the reservoir unit (2) comprises a lateral opening (32) in an outer surface such that the protrusion (73) can be inserted into the opening to lock the reservoir (5) when the arm (70) is in the locking position.

5. Safety mechanism according to claim 4, wherein the connecting end is pivotable supported by the housing (10).

6. Safety mechanism according to any of claims 1 to 5, wherein the drive unit (3) includes a carrier (60) adapted to releasably hold a rim or flange of the reservoir (5) such that the reservoir unit (2) is coupled to the drive unit (3).

7. Safety mechanism according to claim 6, wherein the carrier (60) is movable along the longitudinal axis to move the reservoir unit (2) via reservoir (5) relative to the housing (10) between an extended position and a retracted position.

8. Safety mechanism according to claim 7, comprising a carrier spring member (31) adapted to move the carrier relative to the housing (10).

9. Safety mechanism according to any of claims 1 to 8, wherein the drive is an electric motor (91) adapted to move the plunger rod (40) in dispensing direction.

10. Safety mechanism according to claim 9, wherein the trigger member (50) is operatively coupled to the motor (91), wherein rotating the motor in a first rotational direction causes the trigger member (50) to be moved in the first direction along the longitudinal axis and rotating the motor in a second, counter rotational direction causes the trigger member (50) to be moved in the second direction along the longitudinal axis.

11. Safety mechanism according to any of claims 6 to 8 and further according to claim 9 or 10, wherein the carrier (60) is additionally operatively coupled to the motor (91) and wherein the carrier (60) is movable along the longitudinal axis relative to the housing (10) by the electric motor (91).

12. Safety mechanism according to any of claims 1 to 11, wherein the reservoir unit (2) comprises one single holding member (30) adapted to envelop the reservoir (5) and wherein the reservoir (5) is movable along the longitudinal axis relative to the holding member (30) between a needle covering position in which the needle (7) is covered by the holding member (30) and a retracted position in which the needle (7) protrudes from the holding member (30).

13. Safety mechanism according to any of claims 1 to 12, wherein the reservoir unit (2) comprises a holding element (33) for holding the reservoir (5) and wherein the housing comprises a release element (12) adapted to engage the holding element (33) to release the reservoir (5) upon contact of the holding element (33) with the release element (12).

14. An injection device (1) comprising the safety mechanism according to any of claims 1 to 13 wherein the reservoir is a prefilled syringe (5) and wherein the reservoir unit (2) is a disposable syringe unit adapted to hold the syringe (5) and the drive unit (3) is reusable adapted to be releasably attached to the syringe unit.

15. Method for connecting a reservoir unit (2) to a drive unit (3) of a drug delivery device (1) for dispensing a liquid drug from a reservoir (5), the method comprising the steps of
- Inserting, a proximal end of the reservoir unit (2) into an opening in the drive unit (3);
- Moving a trigger member (50) along a longitudinal axis relative to a drive unit housing (10);
- Moving an arm (70) of the drive unit, by the trigger member movement, from a radially outer release position to a radially inner locking position, in which locking position the reservoir unit (2) is held and locked relative to the drive unit (3) by the arm (70).
